(19) Europäisches Patentamt
European Patent Office
Office européen des brevets

(11) Veröffentlichungsnummer: **0 258 631**
**A2**

(12) # EUROPÄISCHE PATENTANMELDUNG

(21) Anmeldenummer: **87110968.2**

(22) Anmeldetag: **29.07.87**

(51) Int. Cl.⁴: **C07C 131/00** , C07C 153/057 , C07D 307/54 , C07D 317/60 , A01N 37/20 , C07D 213/56 , C07D 263/32 , C07D 263/56 , C07D 277/30 , C07D 277/64 , C07D 307/81

(30) Priorität: **09.08.86 DE 3627072**
**25.03.87 DE 3709780**

(43) Veröffentlichungstag der Anmeldung:
**09.03.88 Patentblatt 88/10**

(84) Benannte Vertragsstaaten:
**AT BE CH DE ES FR GB GR IT LI NL**

(71) Anmelder: **BAYER AG**
**Konzernverwaltung RP Patentabteilung**
**D-5090 Leverkusen 1 Bayerwerk(DE)**

(72) Erfinder: **Jelich, Klaus, Dr.**
**Pahlkestrasse 5**
**D-5600 Wuppertal 1(DE)**
Erfinder: **Gayer, Herbert, Dr.**
**Alfred-Delp-Strasse 4**
**D-4019 Monheim(DE)**
Erfinder: **Krämer, Wolfgang, Dr.**
**Rosenkranz 25**
**D-5093 Burscheid 2(DE)**
Erfinder: **Brandes, Wilhelm, Dr.**
**Eichendorffstrasse 3**
**D-5653 Leichlingen(DE)**
Erfinder: **Hänssler, Gerd, Dr.**
**Am Arenzberg 58 a**
**D-5090 Leverkusen 3(DE)**

(54) **Alkoximinoalyklamide.**

(57) Alkoximinoalkylamide der allgemeinen Formel (I),

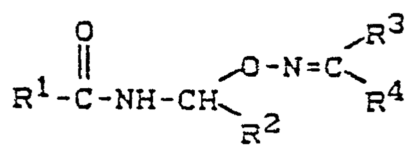

(I)

in welcher
$R^1$ für jeweils gegebenenfalls substituiertes Alkyl, Alkenyl, Aralkyl, Heteroarylalkyl, Aryl oder Heteroaryl steht,
$R^2$ für Cyano, für die Gruppe

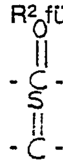

-$NH_2$ oder für die Gruppe
-$NH_2$ steht und

EP 0 258 631 A2

$R^3$ und $R^4$ unabhängig voneinander jeweils für Wasserstoff, für gegebenenfalls substituiertes Alkyl, für Alkenyl oder für Alkinyl stehen, oder gemeinsam mit dem Kohlenstoffatom, an welches sie gebunden sind, für einen gesättigen carbocyclischen Ring stehen,
Verfahren zu deren Herstellung und ihre Verwendung als Schädlingsbekämpfungsmittel.

## Alkoximinoalkylamide

Die Erfindung betrifft neue Alkoximinoalkylamide, mehrere Verfahren zu ihrer Herstellung und ihre Verwendung als Schädlingsbekämpfungsmittel.

Es ist bereits bekannt, daß bestimmte substituierte Amide, wie beispielsweise das 2-(4-Chlorbenzamido)-2-ethoxyacetonitril fungizide Eigenschaften besitzen (vgl. z.B. EP 59 536).

Die Wirksamkeit dieser vorbekannten Verbindungen ist jedoch insbesondere bei niedrigen Aufwandmengen und -konzentrationen nicht in allen Anwendungsbereichen völlig zufriedenstellend.

Es wurden neue Alkoximinoalkylamide der allgemeinen Formel (I),

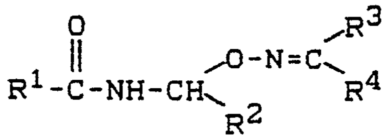

(I)

in welcher
$R^1$ für jeweils gegebenenfalls substituiertes Alkyl, Alkenyl, Aralkyl, Heteroarylalkyl, Aryl oder Heteroaryl steht,
$R^2$ für Cyano, für die Gruppe
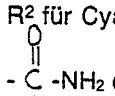
oder für die Gruppe
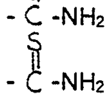
steht und
$R^3$ und $R^4$ unabhängig voneinander jeweils für Wasserstoff, für gegebenenfalls substituiertes Alkyl, für Alkenyl oder für Alkinyl stehen, oder gemeinsam mit dem Kohlenstoffatom, an welches sie gebunden sind, für einen gesättigten carbocyclischen Ring stehen,
gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß die neuen Alkoximinoalkylamide der allgemeinen Formel (I),

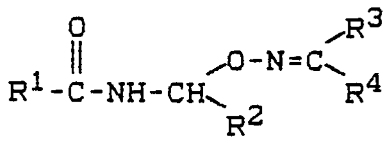

(I)

in welcher
$R^1$ für jeweils gegebenenfalls substituiertes Alkyl, Alkenyl, Aralkyl, Heteroarylalkyl, Aryl oder Heteroaryl steht,
$R^2$ für Cyano, für die Gruppe
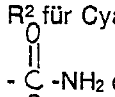
oder für die Gruppe
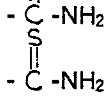
steht und
$R^3$ und $R^4$ unabhängig voneinander jeweils für Wasserstoff, für gegebenenfalls substituiertes Alkyl, für Alkenyl oder für Alkinyl stehen, oder gemeinsam mit dem Kohlenstoffatom, an welches sie gebunden sind, für einen gesättigten carbocyclischen Ring stehen,
erhält, wenn man

(a) bromsubstituierte Amide der allgemeinen Formel (II),

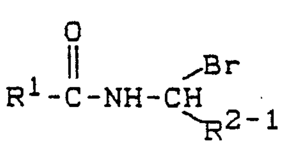

(II)

in welcher
$R^1$ die oben angegebene Bedeutung hat und
$R^{2-1}$ für Cyano oder für eine Carbamoylgruppe steht,
mit Oximen der allgemeinen Formel (III),

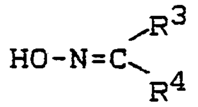

(III)

in welcher
$R^3$ und $R^4$ die oben angegebene Bedeutung haben,
gegebenenfalls in Gegenwart eines Verdünnungsmittels sowie gegebenenfalls in Gegenwart eines Säurebindemittels umsetzt und gegebenenfalls anschließend

(b) die nach Verfahren (a) erhältlichen Alkoximinoalkylamide der allgemeinen Formel (Ia),

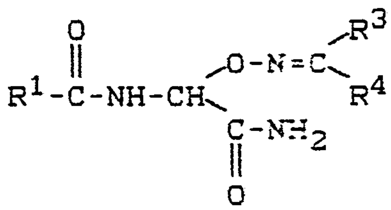

(Ia)

in welcher
$R^1$,$R^3$ und $R^4$ die oben angegebene Bedeutung haben,
gegebenenfalls in Gegenwart eines Verdünnungsmittels und gegebenenfalls in Gegenwart eines Säurebindemittels mit einem wasserabspaltenden Mittel dehydratisiert und gegebenenfalls anschließend

(c) die nach Verfahren (a) oder (b) erhältlichen Alkoximinoalkylamide der allgemeinen Formel (Ib),

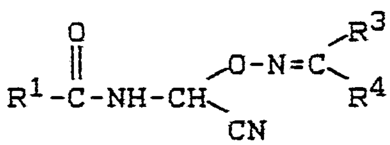

(Ib)

in welcher
$R^1$, $R^3$ und $R^4$ die oben angegebene Bedeutung haben,
mit Schwefelwasserstoff gegebenenfalls in Gegenwart eines Verdünnungsmittels und gegebenenfalls in Gegenwart eines basischen Katalysators umsetzt.

Schließlich wurde gefunden, daß die neuen Alkoximinoalkylamide der allgemeinen Formel (I) fungizide und akarizide Eigenschaften besitzen. Überraschenderweise zeigen die erfindungsgemäßen Alkoximinoalkylamide der allgemeinen Formel (I) bessere fungizide Eigenschaften als die aus dem Stand der Technik bekannten substituierten Amide, wie beispielsweise das N-(Cyano-ethoxy-methyl)-4-chlorobenzamid, welche chemisch und wirkungsmäßig naheliegende Verbindungen sind.

Die erfindungsgemäßen Alkoximinoalkylamide sind durch die Formel (I) allgemein definiert. Bevorzugt sind Verbindungen der Formel (I), bei welchen

$R^1$ für geradkettiges oder verzeigtes Alkyl mit 1 bis 8 Kohlenstoffatomen, für geradkettiges oder verzweigtes Cyanalkyl mit 1 bis 5 Kohlenstoffatomen im Alkylteil, für geradkettiges oder verzweigtes Halogenalkyl mit 1 bis 5 Kohlenstoffatomen und 1 bis 9 Halogenatomen, für jeweils geradkettiges oder verzweigtes Alkoxyalkyl oder Alkylthioalkyl mit jeweils 1 bis 5 Kohlenstoffatomen in den einzelnen Alkylteilen, für geradkettiges oder verzweigtes Alkenyl mit 3 bis 8 Kohlenstoffatomen oder für geradkettiges oder verzweigtes Halogenalkenyl mit 3 bis 8 Kohlenstoffatomen und 1 bis 5 Halogenatomen steht, außerdem für im Arylteil gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil, für im Heteroarylteil gegebenenfalls einfach oder mehrfach, gleich oder verschieden substituiertes Heteroarylalkyl mit 1 bis 4 Kohlenstoffatomen im Alkylteil und 2 bis 9 Kohlenstoffatomen sowie 1 bis 3 Heteroatomen -insbesondere Stickstoff, Sauerstoff und Schwefel -im Heteroarylteil, für gegebenenfalls einfach oder mehrfach, gleich oder verschieden substituiertes Aryl mit 6 bis 10 Kohlenstoffatomen oder für gegebenenfalls einfach oder mehrfach,

gleich oder verschieden substituiertes Heteroaryl mit 2 bis 9 Kohlenstoffatomen und 1 bis 3 Heteroatomen -insbesondere Stickstoff, Sauerstoff und Schwefel -steht, wobei als Aryl-bzw. Heteroaryl substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, Alkylendioxy mit 1 bis 3 Kohlenstoffatomen, Phenyl, Phenoxy, Formyl, Alkanoyl mit 1 bis 5 Kohlenstoffatomen, Alkoxycarbonyl mit 1 bis 5 Kohlenstoffatomen oder Alkoximinoalkyl mit jeweils 1 bis 5 Kohlenstoffatomen in den einzelnen Alkylteilen,

$R^2$ für Cyano, für die Gruppe

$-\overset{\overset{O}{\|}}{C}-NH_2$ oder für die Gruppe

$-\overset{\overset{S}{\|}}{C}-NH_2$ steht und

$R^3$ und $R^4$ unabhängig voneinander jeweils für Wasserstoff, für geradkettiges oder verzweigtes Alkyl mit 1 bis 8 Kohlenstoffatomen, für geradkettiges oder verzweigtes Cyanalkyl mit 1 bis 5 Kohlenstoffatomen, für geradkettiges oder verzweigtes Halogenalkyl mit 1 bis 5 Kohlenstoffatomen und 1 bis 9 Halogenatomen, für jeweils geradkettiges oder verzweigtes Alkoxyalkyl oder Alkylthioalkyl mit jeweils 1 bis 5 Kohlenstoffatomen in den einzelnen Alkylteilen oder für jeweils geradkettiges oder verzweigtes Alkenyl oder Alkinyl mit jeweils 3 bis 7 Kohlenstoffatomen stehen oder

$R^3$ und $R^4$ gemeinsam, mit dem Kohlenstoffatom, an welches sie gebunden sind, für einen gesättigten 5-bis 7-gliedrigen carbocyclischen Ring stehen.

Besonders bevorzugt sind Verbindungen der Formel (I), bei welchen

$R^1$ für geradkettiges oder verzweigtes Alkyl mit 1 bis 5 Kohlenstoffatomen, für Methoxymethyl, für Methylthiomethyl, für Allyl, Butenyl, für jeweils gegebenenfalls ein-bis fünffach gleich oder verschieden im Aryteil substituiertes Benzyl, Phenylethyl, Phenyl oder Napthyl steht oder für jeweils gegebenenfalls in Heteroarylteil ein-bis dreifach gleich oder verschieden substituiertes Heteroarylmethyl oder Heteroaryl steht, wobei als Heteroaromaten jeweils infrage kommen:

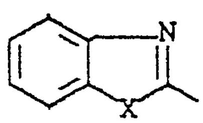 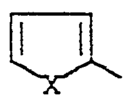 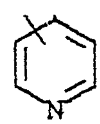 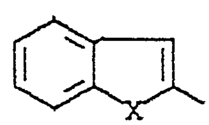 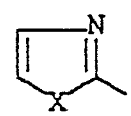

wobei X jeweils für Sauerstoff oder Schwefel steht und als Aryl-bzw. Heteroarylsubstituenten jeweils infrage kommen: Fluor, Chlor, Brom, Iod, Cyano, Nitro, Methyl, Ethyl, n-oder i-Propyl, n-, i-, s-oder t-Butyl, Methoxy, Ethoxy, Methylthio, Trifluromethyl, Trifluormethoxy, Trifluormethylthio, Methylendioxy, Ethylendioxy, Phenyl, Phenoxy, Formyl, Acetyl, Methoxycarbonyl, Ethoxycarbonyl, Methoximinomethyl, Methoximinoethyl und Ethoximinomethyl,

$R^2$ für Cyano oder für eine Thiocarbamoylgruppe steht und

$R^3$ und $R^4$ unabhängig voneinander jeweils für Wasserstoff, für Methyl, Ethyl, n-oder i-Propyl, n-, i-, s-oder t-Butyl, für Allyl, Propenyl, n-oder i-Butenyl oder Propargyl stehen oder gemeinsam mit dem Kohlenstoffatom, an welches sie gebunden sind, für einen Cylcopentyl-oder Cyclohexylrest stehen.

Ganz besonders bevorzugt sind Verbindungen der Formel (I), bei welchen

$R^1$ für jeweils gegebenenfalls ein-bis dreifach, gleich oder verschieden substituiertes Phenyl, Pyridyl, Benzoxazolyl, Benzthiazolyl, Furyl oder Thienyl steht, wobei als Substituenten jeweils infrage kommen: Methyl, Ethyl, Methoxy, Fluor, Chlor, Brom, Methylendioxy oder Ethylendioxy,

$R^2$ für Cyano steht und

$R^3$ und $R^4$ unabhängig voneinander jeweils für Wasserstoff, Methyl oder Ethyl stehen.

Im einzelnen seien außer den bei den Herstellungsbeispielen genannten Verbindungen die folgenden Alkoximinoalkylamide der allgemeinen Formel (I) genannt:

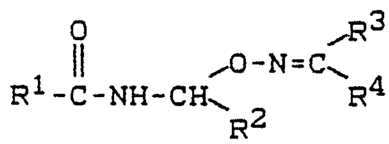 (I)

| $R^1$ | $R^2$ | 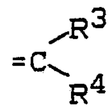 |
|---|---|---|
| 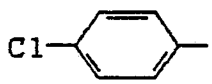 | CN | 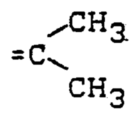 |
| 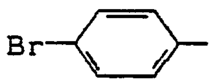 | CN | $=CH_2$ |
| 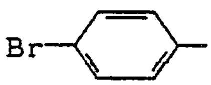 | CN | $=CH-CH_3$ |
| 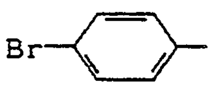 | CN | 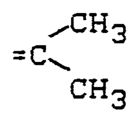 |
| 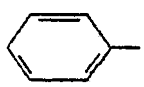 | CN | $=CH_2$ |
| 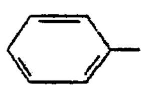 | CN | $=CH-CH_3$ |
| 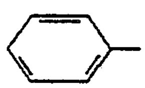 | CN | 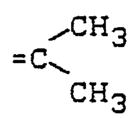 |
| 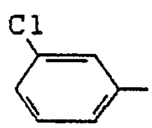 | CN | $=CH_2$ |
| 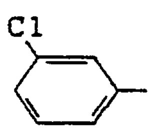 | CN | $=CH-CH_3$ |
| 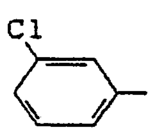 | CN | 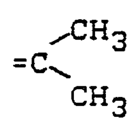 |

| $R^1$ | $R^2$ | 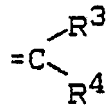 |
|---|---|---|
| 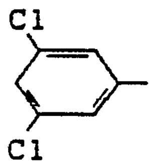 | CN | $=CH_2$ |
| 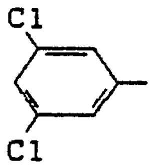 | CN | $=CH-CH_3$ |
| 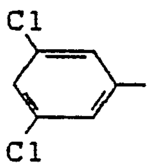 | CN | 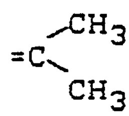 |
| 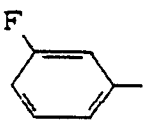 | CN | $=CH_2$ |
| 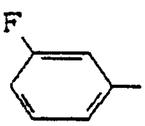 | CN | $=CH-CH_3$ |
| 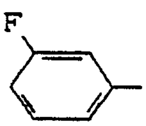 | CN | 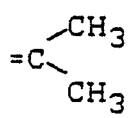 |
| 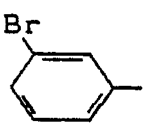 | CN | $=CH_2$ |
| 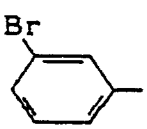 | CN | $=CH-CH_3$ |
| 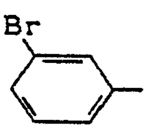 | CN | 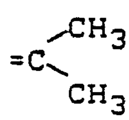 |

| $R^1$ | $R^2$ | 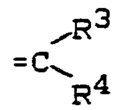 |
|---|---|---|
| 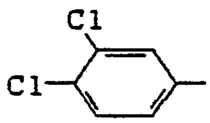 | CN | $=CH_2$ |
| 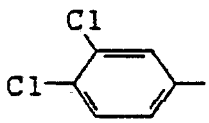 | CN | $=CH-CH_3$ |
| 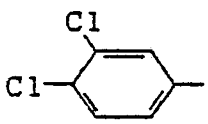 | CN | 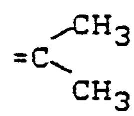 |
| 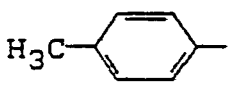 | CN | $=CH_2$ |
| 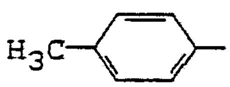 | CN | $=CH-CH_3$ |
| 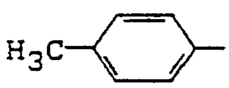 | CN | 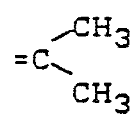 |
| 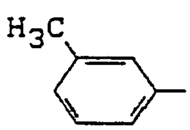 | CN | $=CH_2$ |
| 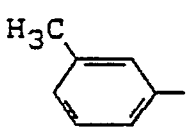 | CN | $=CH-CH_3$ |
| 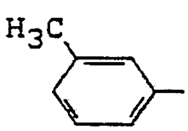 | CN | 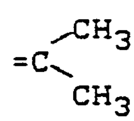 |

| $R^1$ | $R^2$ | 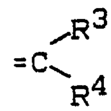 |
|---|---|---|
| 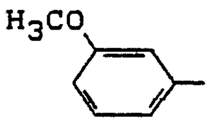 | CN | $=CH_2$ |
| 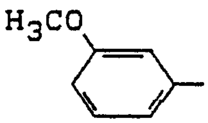 | CN | $=CH-CH_3$ |
| 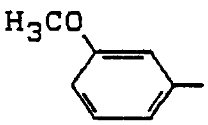 | CN | 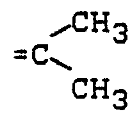 |
| 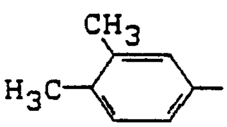 | CN | $=CH_2$ |
| 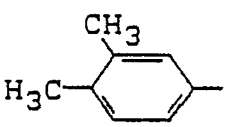 | CN | $=CH-CH_3$ |
| 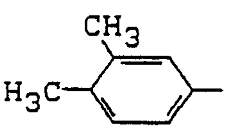 | CN | 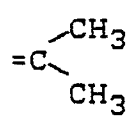 |
| 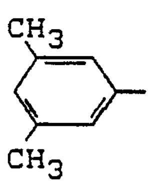 | CN | $=CH_2$ |
| 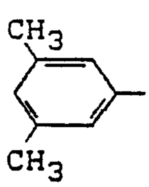 | CN | $=CH-CH_3$ |
| 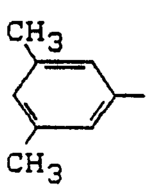 | CN | 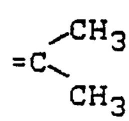 |

| $R^1$ | $R^2$ | 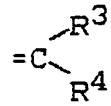 |
| --- | --- | --- |
| 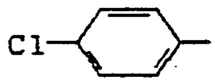 | 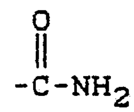 | $=CH-CH_3$ |
| 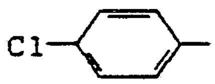 | 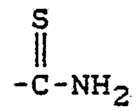 | $=CH-CH_3$ |
| 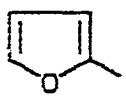 | CN | $=CH-CH_3$ |
| 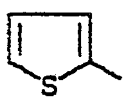 | CN | $=CH-CH_3$ |
| 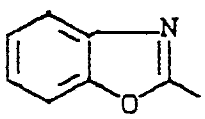 | CN | $=CH-CH_3$ |
| 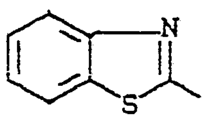 | CN | $=CH-CH_3$ |
| $(CH_3)_2CH-CH_2-$ | CN | $=CH-CH_3$ |
| 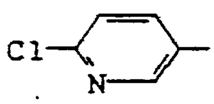 | CN | $=CH-CH_3$ |
| 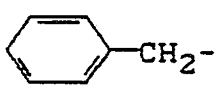 | CN | $=CH-CH_3$ |
| 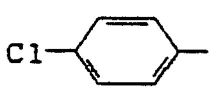 | CN | $=CH-C_2H_5$ |
| 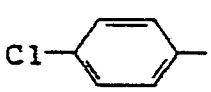 | CN | 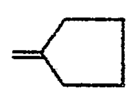 |
| 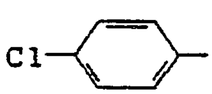 | CN | $=CH-CH=CH-CH_3$ |

Verwendet man beispielsweise N-(Brom-cyano-methyl)-4-chlorobenzamid und Acetaldehydoxim als Ausgangsstoffe, so läßt sich der Reaktionsablauf des erfindungsgemäßen Verfahrens (a) durch das folgende Formelschema darstellen:

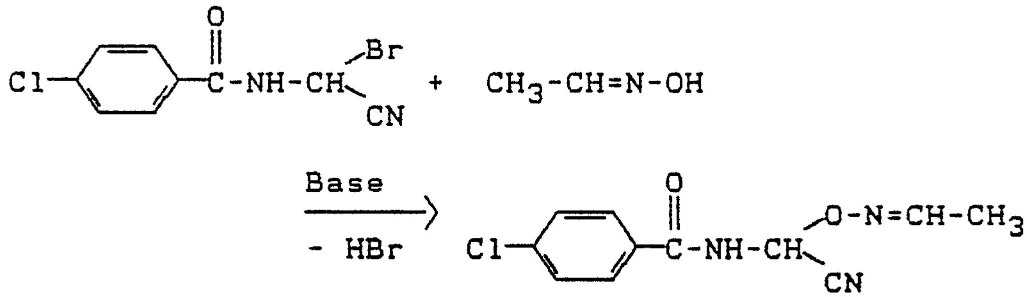


Verwendet man beispielsweise die erfindungsgemäße Verbindung 2-(4-Chlorbenzamido)-2-ethylideniminoxy-acetamid als Ausgangsverbindung und p-Toluolsulfonylchlorid als Dehydratisierungsmittel, so läßt sich der Reaktionsablauf des erfindungsgemäßen Verfahrens (b) durch das folgende Formelschema darstellen:

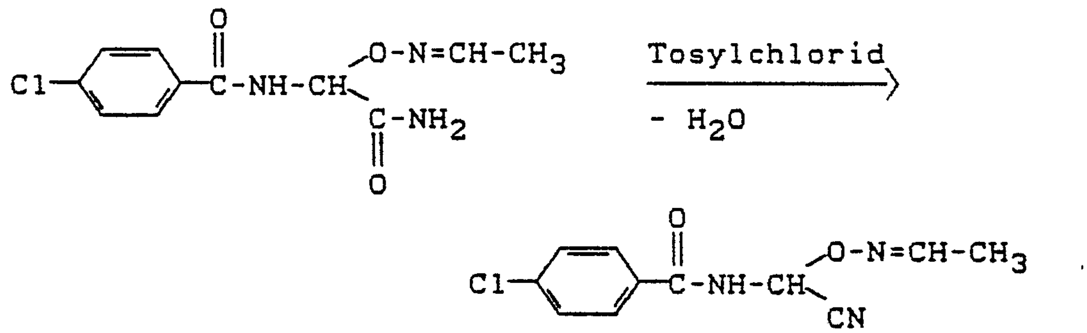


Verwendet man beispielsweise die erfindungsgemäße Verbindung 2-(4-Chlorbenzamido)-2-ethylideniminoxyacetonitril und Schwefelwasserstoff als Ausgangsstoffe, so läßt sich der Reaktionsablauf des erfindungsgemäßen Verfahrens (c) durch das folgende Formelschema darstellen:

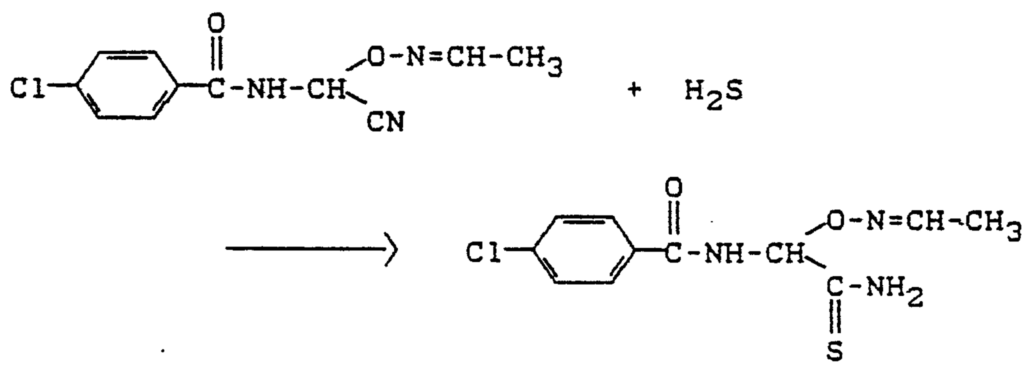

Die zur Durchführung des erfindungsgemäßen Verfahrens (a) als Ausgangsstoffe benötigten bromsubstituierten Amide sind durch die Formel (II) allgemein definiert. In dieser Formel (II) steht $R^1$ vorzugsweise für diejenigen Reste, die bereits im Zusammenhang mit der Beschreibung der erfindungsgemäßen Stoffe der Formel (I) für diesen Substituenten genannt wurden. $R^{2-1}$ steht vorzugsweise für Cyano oder für die Carbamoylgruppe.

Die bromsubstituierten Amide der Formel (II) sind bekannt (vgl. EP 59 536 und EP 135 304).

Die zur Durchführung des erfindungsgemäßen Verfahrens (a) weiterhin als Ausgangsstoffe benötigten Oxime sind durch die Formel (III) allgemein definiert. In dieser Formel (III) stehen $R^3$ und $R^4$ für diejenigen Reste, die bereits im Zusammenhang mit der Beschreibung der erfindungsgemäßen Stoffe der Formel (I) für diese Substituenten genannt wurden.

Die Oxime der Formel (III) sind allgemein bekannte Verbindungen der organischen Chemie.

Die zur Durchführung des erfindungsgemäßen Verfahrens (b) als Ausgangsstoffe benötigten Alkoximinoalkylamide sind durch die Formel (Ia) allgemein definiert. In dieser Formel (Ia) stehen $R^1$, $R^3$ und $R^4$ für diejenigen Reste, die bereits im Zusammenhang mit der Beschreibung der erfindungsgemäßen Stoffe der Formel (I) für diese Substituenten genannt wurden.

Die Alkoximinoalkylamide der Formel (Ia) sind erfindungsgemäße Verbindungen und erhältlich mit Hilfe des erfindungsgemäßen Verfahrens (a).

Die zur Durchführung des erfindungsgemäßen Verfahrens (c) als Ausgangsstoffe benötigten Alkoximinoalkylamide sind durch die Formel (Ib) allgemein definiert. In dieser Formel (Ib) stehen $R^1$, $R^3$ und $R^4$ für diejenigen Reste, die bereits im Zusammenhang mit der Beschreibung der erfindungsgemäßen Stoffe der Formel (I) für diese Substituenten genannt wurden.

Die Alkoximinoalkylamide der Formel (Ib) sind erfindungsgemäße Verbindungen und erhältlich mit Hilfe der erfindungsgemäßen Verfahren (a) und (b).

Als Verdünnungsmittel zur Durchführung des erfindungsgemäßen Verfahrens (a) kommen inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykol dimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird vorzugsweise in Gegenwart eines geeigneten Säurebindemittels durchgeführt. Hierzu gehören vorzugsweise tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindunsgemäßen Verfahrens (a) in einem größeren Bereich variiert werden. Im allgemeinen arbeitet man bei Temperaturen zwischen -50° C und +100° C, vorzugsweise bei Temperaturen zwischen -30° C und +50° C.

Zur Durchführung des erfindungsgemäßen Verfahrens (a) setzt man pro Mol an bromsubstituiertem Amid der Formel (II) im allgemeinen 1.0 bis 3.0 Mol, insbesondere 1.0 bis 1.2 Mol an Oxim der Formel (III) und 1.0 bis 3.0 Mol, insbesondere 1.0 bis 2.0 Mol an Säurebindemittel ein.

In einer bevorzugten Durchführungsform stellt man die als Ausgangsstoffe verwendeten bromsubstituierten Amide der Formel (II) in einer vorgelagerten Reaktion direkt im Reaktionsgefäß her, indem man die entsprechenden Cyanmethylamide der Formel (IV),

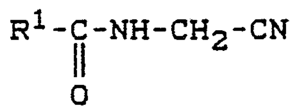

(IV)

in welcher

$R^1$ die oben angegebene Bedeutung hat,

mit einem geeigneten Bromierungsmittel, wie beispielsweise mit elementarem Brom, in einem geeigneten Verdünnungsmittel, wie beispielsweise Essigsäure oder Essigester, und gegebenenfalls in Gegenwart eines geeigneten sauren Katalysators, wie beispielsweise Bromwasserstoffsäure, bei Temperaturen zwischen -20° C und + 20° C bromiert und direkt anschließend in 'Eintopfverfahren' gemäß dem erfindungsgemäßen Verfahren (a) weiter umsetzt.

Je nach Wahl des Verdünnungsmittels und in Abhängigkeit von der Säurekonzentration erhält man bei dieser vorgelagerten Bromierungsreaktion entweder eine gleichzeitige Hydratisierung der Nitrilgruppe zu den entsprechenden Amiden der Formel (IIa),

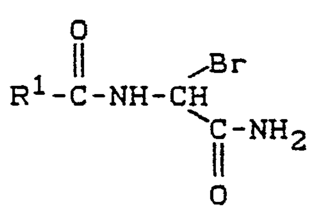

(IIa)

in welcher
$R^1$ die oben angegebene Bedeutung hat,
oder Amide der Formel (II b),

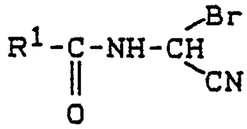

(II b)

in welcher
$R^1$ die oben angegebene Bedeutung hat,
bei denen die Nitrilgruppe als solche erhalten bleibt (vergl. EP 59 536, EP 135 304 sowie die Herstellungsbeispiele).

Die Durchführung des erfindungsgemäßen Verfahrens (a) sowie die Aufarbeitung und Isolierung der Reaktionsprodukte der Formel (Ia) oder (Ib) erfolgt danach in Analogie zu bekannten Verfahren (vergl. EP 59 536).

Als Dehydratisierungsmittel zur Durchführung des erfindungsgemäßen Verfahrens (b) kommen alle üblichen wasserabspaltenden Mittel infrage. Mit besonderem Vorteil verwendet man Säurechloride oder Säureanhydride, wie beispielsweise Trifluoracetanhydrid, p-Toluolsulfonylchlorid, Trichloracetylchlorid, Methansulfonylchlorid, Titantetrachlorid oder Phosphoroxychlorid.

Als Verdünnungsmittel zur Durchführung des erfindungsgemäßen Verfahren (b) kommen ebenfalls inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Pyridin, Petrolether, Hexan, Cyclohexan, Dichlormethan, Chloroform, Tetrachlorkohlenstoffe, Ether, wie Diethylether, Dioxan, Tetrahydrofuran oder Ethylenglykoldimethyl-oder -diethylether, Nitrile, wie Acetonitril oder Propionitril oder Amide, wie Dimethylformamid.

Das erfindungsgemäße Verfahren (b) wird gegebenenfalls in Gegenwart eines geeigneten Säurebindemittels durchgeführt. Als solche eignen sich insbesondere tertiäre Amine, wie N-Methylmorpholin, Triethylamin, N,N-Dimethylanilin, Pyridin, N,N-Dimethylaminopyridin, Diazabicyclooctan (DABCO), Dizabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20° C und +80° C, vorzugsweise bei Temperaturen zwischen 0° C und +40° C.

Zur Durchführung des erfindungsgemäßen Verfahrens (b) setzt man pro Mol Alkoximinoalkylamid der Formel (Ia) im allgemeinen 1.0 bis 5.0 Mol, vorzugsweise 1.0 bis 3.0 Mol an Dehydratisierungsmittel ein. Die Reaktionsdurchführung, Aufarbeitung und Isolierung der Reaktionsprodukte der Formel (Ib) erfolgt in Analogie zu bekannten Verfahren (vergl. EP 59 536).

Als Verdünnungsmittel zur Durchführung des erfindungsgemä ßen Verfahrens (c) kommen ebenfalls inerte organische Lösungsmittel infrage. Hierzu gehören insbesondere die bei Verfahren (a) genannten organischen Lösungsmittel. Mit besonderem Vorteil verwendet man Toluol oder Pyridin als Verdünnungsmittel.

Das erfindungsgemäße Verfahren (c) wird vorzugsweise in Gegenwart eines basischen Katalysators durchgeführt. Als solche eignen sich insbesondere die bei Verfahren (a) aufgezählten tertiären Amine. Mit besonderem Vorzug verwendet man Triethylamin.

Die Reaktionstemperaturen können bei der Durchführung des erfindungsgemäßen Verfahrens (c) in einem größeren Bereich variiert werden. Im allgemeinen arbeitet man bei Temperaturen zwischen -20° C und +30° C, vorzugsweise bei Temperaturen zwischen 0° C und +20° C.

Zur Durchführung des erfindungsgemäßen Verfahrens (c) setzt man pro Mol Alkoximinoalkylamid der Formel (Ib) im allgemeinen 1.0 bis 5.0 Mol, insbesondere 1.0 bis 2.0 Mol an basischen Katalysator ein und leitet gasförmigen Schwefelwasserstoff durch die Reaktionslösung. Die Aufarbeitung und Isolierung der Reaktionsprodukte der Formel (I) erfolgt durch Abfiltrieren von ausgefallenem festem Produkte oder durch Entfernen der flüchtigen Bestandteile der Reaktionsmischung durch Eindampfen (vgl. auch EP 59 536).

Die erfindungsgemäßen Wirkstoffe weisen eine starke mikrobizide und akarizide Wirkung auf und können zur Bekämpfung von unerwünschten Mikroorganismen und Milben praktisch eingesetzt werden. Die Wirkstoffe sind z.B. für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora.infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccina-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die Wirkstoffe eignen sich auch zur Bekämpfung von tierischen Schädlingen, vorzugsweise Arthropoden, insbesondere Insekten und Spinnentieren, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum,

Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia supp,. Chrysomyia spp., Cuterebra spp., Gastrophilus spp., Hyppobosca spp., Stomoxys spp., Oestrus spp., Hypoderma spp., Tabanus spp, Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B.Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Dabei lassen sich die erfindungsgemäßen Wirkstoffe mit besonders gutem Erfolg zur Bekämpfung von Gemüsekrankheiten, wie beispielsweise gegen den Erreger der Tomatenbraunfäule (Phytophthora infestans) sowie zur Bekämpfung von Getreidekrankheiten, weiterhin beispielsweise gegen der Erreger der Reiskrankheit Pyricularia oryzae oder gegen Pythium-Arten insbesondere bei der Saatgutbeizung einsetzen, außerdem gegen pflanzenschädigende Milben (Tetranychus).

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streck mitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie

synthetische Granulate aus anorganischen und organi schen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Der Wirkstoffgehalt der aus den handelsüblichen Formulierungen bereiteten Anwendungsformen zur Bekämpfung von tierischen Schädlingen kann in weiten Bereichen variieren. Die Wirkstoffkonzentration der Anwendungsformen kan von 0,0000001 bis 95 Gew.-% Wirkstoff, vorzugsweise zwischen 0,0001 und 1 Gew.-% liegen.

Herstellungsbeispiele

Beispiel 1:

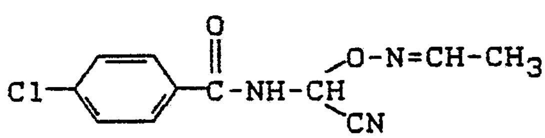

(Verfahren a)

Zu 5,07 g (0.0261 Mol) 4-Chlorbenzoylamino-acetonitril in 250 ml absolutem Essigester gibt man bei 0° C einige Tropfen Brom und startet die Reaktion durch Zugabe einiger Tropfen einer 33%igen Lösung von Bromwasserstoff in Eisessig, wobei sich die Reaktionsmischung entfärbt. Anschließend gibt man ebenfalls bei 0° C das restliche Brom [insgesamt 4,2 g (0,0262 Mol)] langsam zu, kühlt nach beendeter Zugabe auf -25° C ab und gibt in einer Portion ein Gemisch aus 1,55 g (0.0262 Mol) Acetaldehydoxim und 5,3g (0.0523 Mol) Triethylamin in 50 ml absolutem Essigester zu, wobei sich die Reaktionsmischung auf -10° C erwärmt

und Triethylaminhydrochlorid ausfällt. Man rührt 15 Minuten bei 0° C, filtriert, engt das Filtrat ein und reinigt den öligen Rückstand durch Chromatographie an Kieselgel (Laufmittel: Dichlormethan).
Man erhält 1,3 g (20 % der Theorie) an 2-(4-Chlorbenzoylamino)-2-ethylideniminoxy-acetonitril als E/Z-Gemisch vom Schmelzpunkt 100° C.

In entsprechender Weise und gemäß den allgemeinen Angaben zur Herstellung erhält man die folgenden Alkoximinoalkylamide der allgemeinen Formel (I):

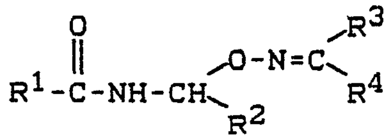

(I)

| Bsp.Nr. | $R^1$ | $R^2$ | 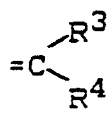 | Schmelz-punkt° C |
|---|---|---|---|---|
| 2 | 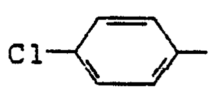 | CN | $=CH_2$ | 129 |
| 3 | 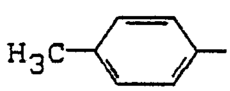 | CN | $=CH-CH_3$ | $^1H$-NMR ($CDCl_3$) $\delta$=7,05ppm |
| 4 | 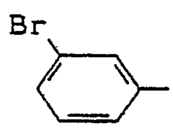 | CN | $=CH_2$ | 115 |
| 5 | 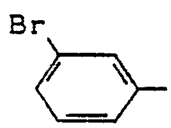 | CN | $=CH-CH_3$ | 96 |
| 6 | 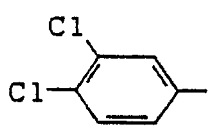 | CN | $=CH_2$ | 130 |
| 7 | 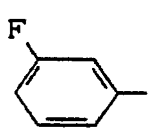 | CN | $=CH_2$ | 127 |
| 8 | 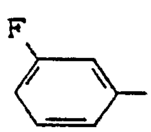 | CN | $=CH-CH_3$ | 152 |

| Bsp.Nr. | $R^1$ | $R^2$ | 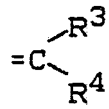 | Schmelz-punkt° C |
|---|---|---|---|---|
| 9 | 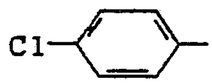 | CN | 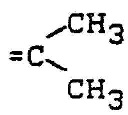 | 104 |
| 10 | 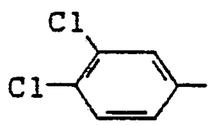 | CN | $=CH-CH_3$ | $^1$H-NMR*): 1,95 |
| 11 | 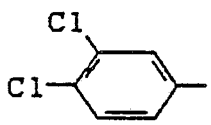 | CN | 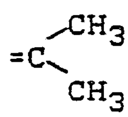 | 116 |
| 12 | 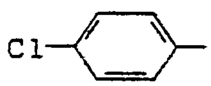 | CN | $=CH-CH=CH_2$ | 110 |
| 13 | 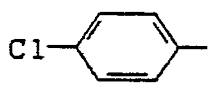 | CN | $=CH-CH=CH-CH_3$ | 112 |
| 14 | 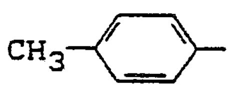 | CN | $=CH-CH=CH-CH_3$ | 117 |
| 15 | 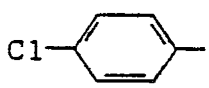 | CN | $=CH-C_2H_5$ | $^1$H-NMR*): 6,7 |
| 16 | 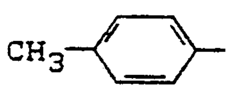 | CN | 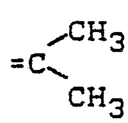 | 129 |
| 17 | 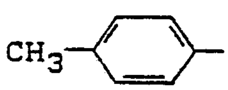 | CN | $=CH_2$ | $^1$-NMR*): 6,7 |
| 18 | 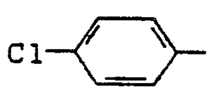 | CN | $=CH-CH=CH_2$ | $^1$-NMR*): 6,7 |

| Bsp.Nr. | $R^1$ | $R^2$ | 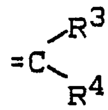 | Schmelz-punkt° C |
|---|---|---|---|---|
| 19 | 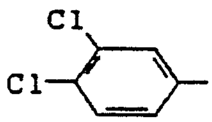 | CN | $=CH-C_2H_5$ | 108 |
| 20 | 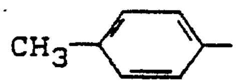 | CN | $=CH-CH=CH_2$ | 112 |
| 21 | 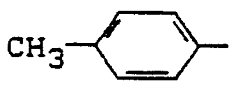 | CN | $=CH-C_2H_5$ | 108 |
| 22 | 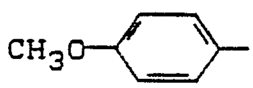 | CN | $=CH-CH_3$ | $^1$H-NMR*): 1,9 |
| 23 | 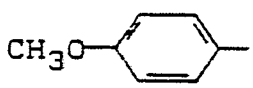 | CN | 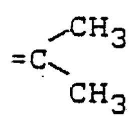 | 95 |
| 24 | 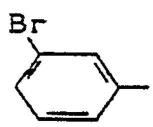 | CN | 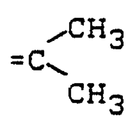 | 121 |
| 25 | 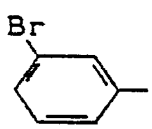 | CN | $=CH-C_2H_5$ | 95 |
| 26 | 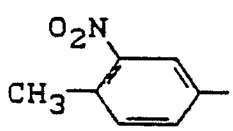 | CN | 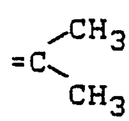 | 130 |
| 27 | 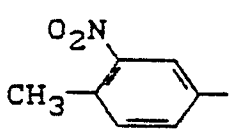 | CN | $=CH-C_2H_5$- | $^1$-NMR*): 6,7 |

| Bsp.Nr. | $R^1$ | $R^2$ | 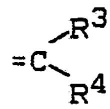 | Schmelzpunkt° C |
|---|---|---|---|---|
| 28 | 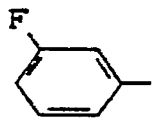 | CN | $=CH-CH=CH_2$ | $^1$-NMR*):6,7 |
| 29 | 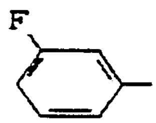 | CN | 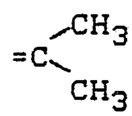 | $^1$-NMR*):1,95 |
| 30 | 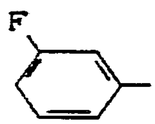 | CN | $=CH-C_2H_5$ | $^1$-NMR*):6,7 |
| 31 | 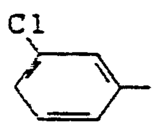 | CN | 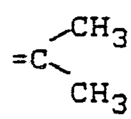 | 94 |
| 32 | 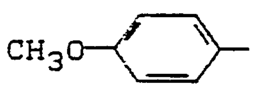 | CN | $=CH-CH=CH_2$ | $^1$-NMR*):3,8 |
| 33 | 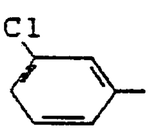 | CN | $=CH-CH=CH_2$ | 106 |
| 34 | 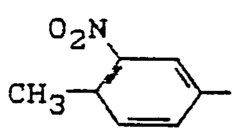 | CN | $=CH_2$ | 134 |
| 35 | 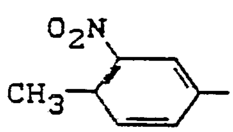 | CN | $=CH-CH_3$ | 99 |
| 36 | 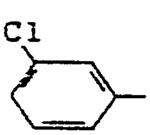 | CN | $=CH-CH_3$ | 92 |

| Bsp.Nr. | $R^1$ | $R^2$ | 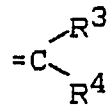 | Schmelz-punkt° C |
|---|---|---|---|---|
| 37 | 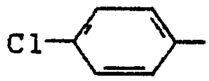 | CN | 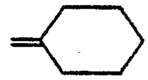 | 138 |
| 38 | 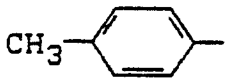 | CN | 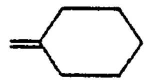 | $^1H$-NMR*): 6,75 |
| 39 | 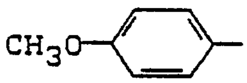 | CN | $=CH-C_2H_5$ | $^1H$-NMR*): 6,7 |
| 40 | 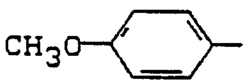 | CN | 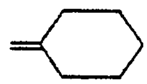 | $^1H$-NMR*): 3,85 |
| 41 | 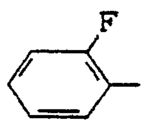 | CN | $=CH_2$ | 84 |
| 42 | 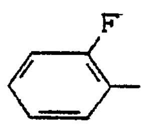 | CN | $=CH-CH_3$ | 68 |
| 43 | 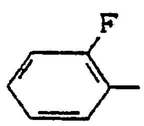 | CN | 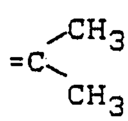 | 56 |
| 44 | 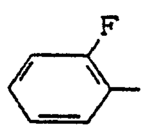 | CN | $=CH-C_2H_5$ | 82 |
| 45 | 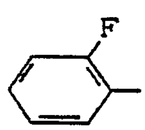 | CN | $=CH-CH=CH_2$ | 73 |
| 46 | 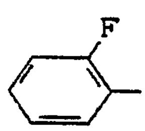 | CN | 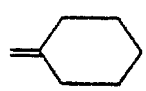 | $^1H$-NMR*): 6,8 |

| Bsp.Nr. | $R^1$ | $R^2$ | 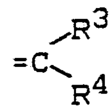 | Schmelz-punkt° C |
|---|---|---|---|---|
| 47 | 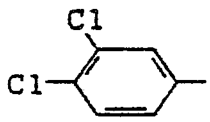 | CN | 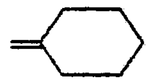 | 116 |
| 48 | 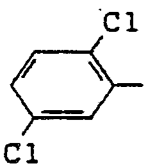 | CN | 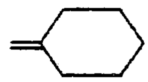 | $^1$H-NMR*): 6,7 |
| 49 | 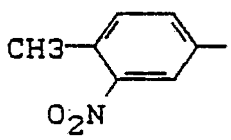 | CN | 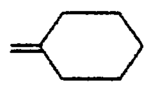 | $^1$H-NMR*): 8,0 |
| 50 | 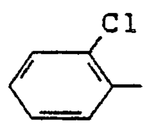 | CN | 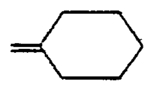 | $^1$H-NMR*): 6,7 |
| 51 | 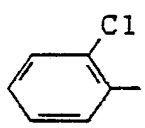 | CN | 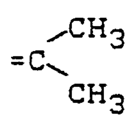 | $^1$H-NMR*): 6,7 |
| 52 | 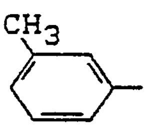 | CN | 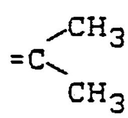 | $^1$H-NMR*): 1,9 |
| 53 | 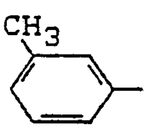 | CN | 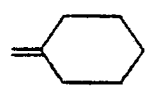 | $^1$H-NMR*): 2,45 |
| 54 | 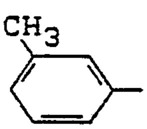 | CN | $=CH-CH_3$ | $^1$H-NMR*): 1,95 |
| 55 | 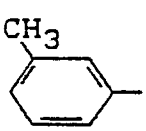 | CN | $=CH-CH=CH_2$ | $^1$H-NMR*): 2,45 |

| Bsp.Nr. | $R^1$ | $R^2$ | $=C{<}^{R^3}_{R^4}$ | Schmelzpunkt° C |
|---|---|---|---|---|
| 56 | 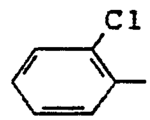 | CN | $=CH-CH_3$ | $^1$H-NMR*): 1,95 |
| 57 | 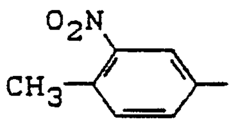 | CN | $=CH-CH=CH_2$ | $^1$H-NMR*): 6,8 |
| 58 | 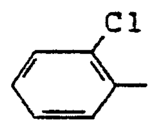 | CN | $=CH-CH-CH_2$ | $^1$H-NMR*): 6,75 |
| 59 | 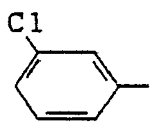 | CN | $=CH-C_2H_5$ | 83 |
| 60 | 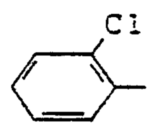 | CN | $=CH-C_2H_5$ | $^1$H-NMR*): 6,7 |
| 61 | 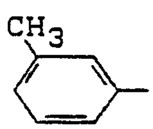 | CN | $=CH-C_2H_5$ | $^1$H-NMR*): 6,7 |
| 62 | 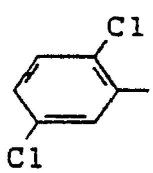 | CN | $=C{<}^{CH_3}_{CH_3}$ | 93 |
| 63 | 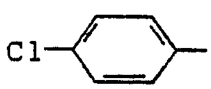 | $-\overset{S}{\overset{\|}{C}}-NH_2$ | $=CH-CH_3$ | 182 |

| Bsp.Nr. | $R^1$ | $R^2$ | 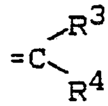 | Schmelzpunkt° C |
|---|---|---|---|---|
| 64 | 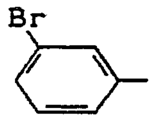 | 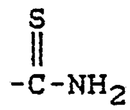 | =CH-$CH_3$ | $^1$H-NMR*): 1,8 |
| 65 | 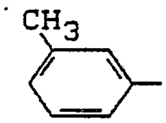 | 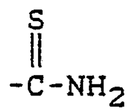 | =CH-$CH_3$ | $^1$H-NMR*): 1,85 |
| 66 | 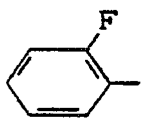 | 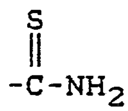 | =CH-$CH_3$ | 134 |
| 67 | 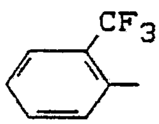 | CN | =CH-$CH_3$ | $^1$H-NMR*): 1,9 |
| 68 | 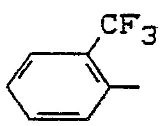 | CN | 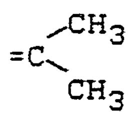 | 98 |
| 69 | 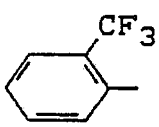 | CN | 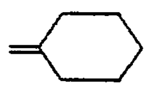 | $^1$H-NMR*): 6,7 |
| 70 | 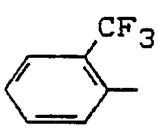 | CN | =CH-$C_2H_5$ | 78-79 |
| 71 | 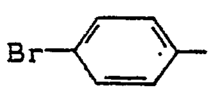 | CN | = CH-$CH_3$ | 108 |
| 72 | 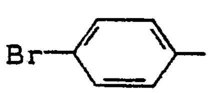 | CN | = 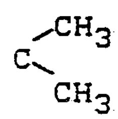 | $^1$H-NMR*): 1,95 |

| Bsp.Nr. | $R^1$ | $R^2$ | 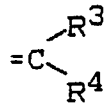 | Schmelzpunkt° C |
|---|---|---|---|---|
| 73 | 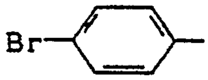 | CN | 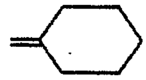 | 150 |
| 74 | 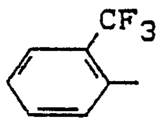 | 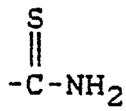 | $=CH-CH_3$ | 141-143 |
| 75 | 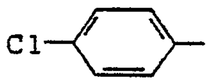 | 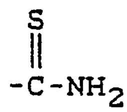 | 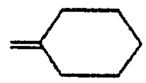 | 133-135 |
| 76 | 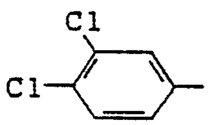 | 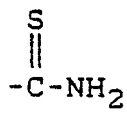 | $=CH-CH_3$ | 157 (Zers.) |
| 77 | 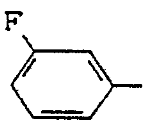 | 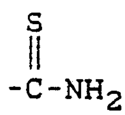 | $=CH-CH_3$ | 155 (Zers.) |
| 78 | 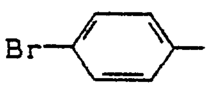 | 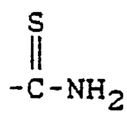 | 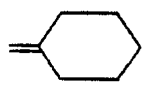 | 133-134 (Zers.) |
| 79 | 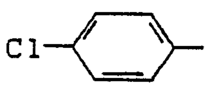 | 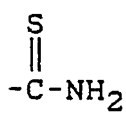 | 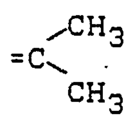 | 136-138 (Zers.) |
| 80 | 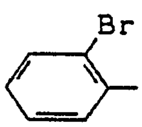 | CN | $=CH-CH_3$ | $^1$H-NMR*): 1,85 |
| 81 | 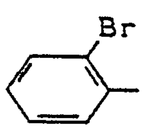 | CN | 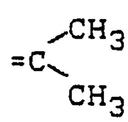 | 88-89 |

| Bsp.Nr. | $R^1$ | $R^2$ | 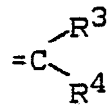 | Schmelzpunkt° C |
|---|---|---|---|---|
| 82 | 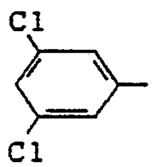 | CN | $=CH-CH_3$ | 162-164 |
| 83 | 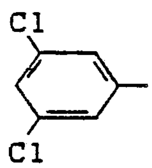 | CN | 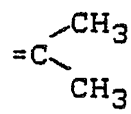 | 101-102 |
| 84 | 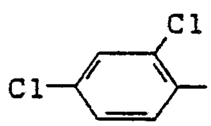 | CN | $=CH-CH_3$ | 106-108 |
| 85 | 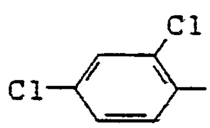 | CN | 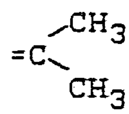 | $^1H$-NMR*): 1,95 |
| 86 | 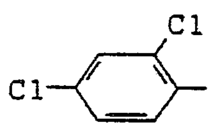 | CN | $=C-C_2H_5$ | $^1H$-NMR*): 6,65 |
| 87 | 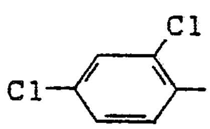 | CN | 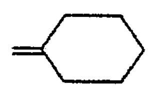 | 113-114 |
| 88 | 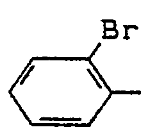 | CN | $=CH-C_2H_5$ | $^1H$-NMR*): 6,65 |
| 89 | 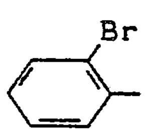 | CN | 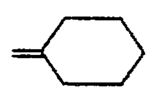 | 110 |

| Bsp.Nr. | $R^1$ | $R^2$ | 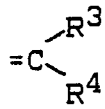 | Schmelzpunkt° C |
|---|---|---|---|---|
| 90 | 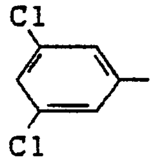 | CN | 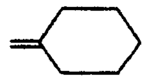 | $^1$H-NMR*): 6,7 |
| 91 | 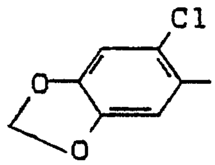 | CN | =CH-$CH_3$ | 103-105 |
| 92 | 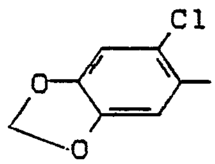 | CN | 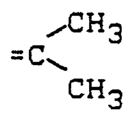 | 142 |
| 93 | 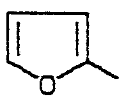 | CN | =CH-$CH_3$ | 89 |
| 94 | 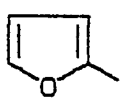 | CN | 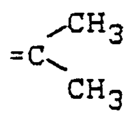 | 77-79 |
| 95 | 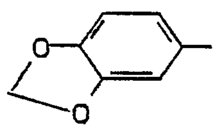 | CN | 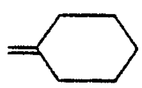 | 116-118 |
| 96 | 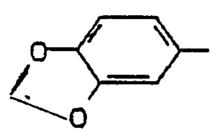 | 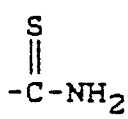 | 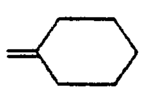 | 132 |
| 97 | 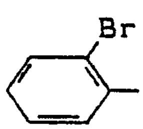 | 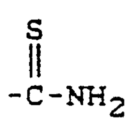 | 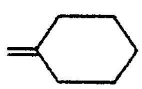 | 135 |

| Bsp.Nr. | $R^1$ | $R^2$ | 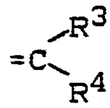 | Schmelz-punkt° C |
|---|---|---|---|---|
| 98 | 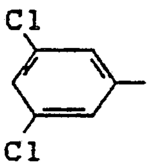 | 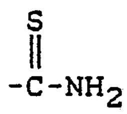 | 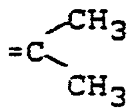 | 200 |
| 99 | 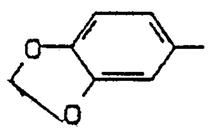 | 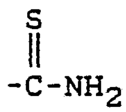 | 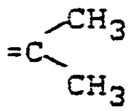 | 143-144 |
| 100 | 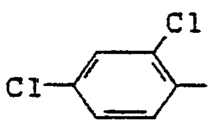 | 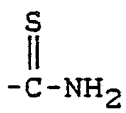 | $=CH-CH_3$ | 139 |
| 101 | 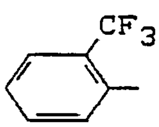 | 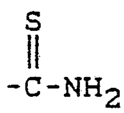 | 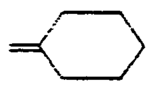 | $^1$H-NMR*): 6,15 |
| 102 | 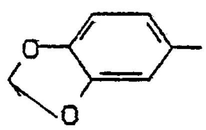 | 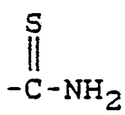 | $=CH-CH_3$ | 137-139 |
| 103 | 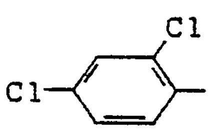 | 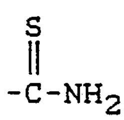 | 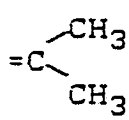 | 144 |
| 104 | 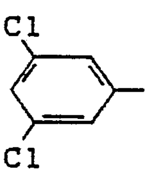 | 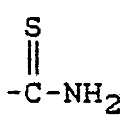 | 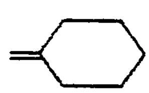 | 142 |
| 105 | 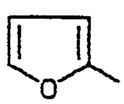 | 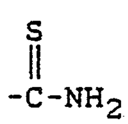 | 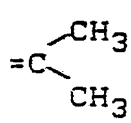 | 144-146 |

| Bsp.Nr. | $R^1$ | $R^2$ | 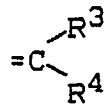 | Schmelz-punkt° C |
|---|---|---|---|---|
| 106 | 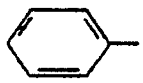 | CN | $=CH-CH_3$ | $^1H$-NMR*): 1,9 |
| 107 | 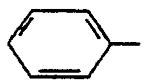 | CN | 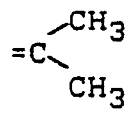 | $^1H$-NMR*): 1,9 |
| 108 | 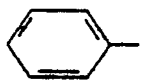 | CN | 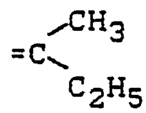 | $^1H$-NMR*): 6,75 |
| 109 | 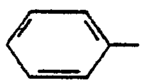 | CN | 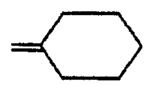 | $^1H$-NMR*): 6,75 |
| 110 | 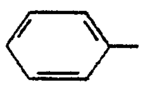 | CN | $=CH-C_2H_5$ | $^1H$-NMR*): 6,75 |
| 111 | 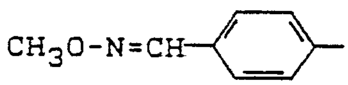 | CN | $=CH-CH_3$ | $^1H$-NMR*): 1,9 |
| 112 | 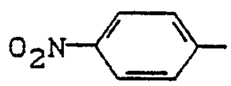 | CN | $=CH_2$ | 152 |
| 113 | 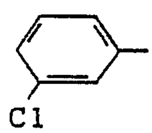 | CN | $=CH_2$ | 108-110 |
| 114 | 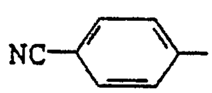 | CN | $=CH_2$ | 180 |
| 115 | 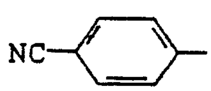 | CN | $=CH-CH_3$ | 160 |
| 116 | 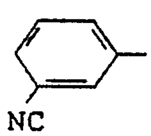 | CN | $=CH_2$ | 146-148 |

| Bsp.Nr. | $R^1$ | $R^2$ | $=C<^{R^3}_{R^4}$ | Schmelzpunkt° C |
|---|---|---|---|---|
| 117 | 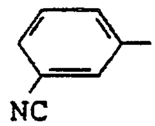 | CN | $=CH-CH_3$ | 142-144 |
| 118 | 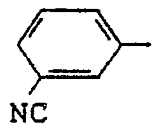 | CN | $=CH-C(CH_3)_3$ | $^1$H-NMR*): 1,12 |
| 119 | 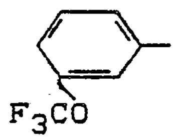 | CN | $=CH_2$ | $^1$H-NMR*): 6,75 |
| 120 | 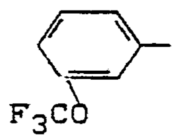 | CN | $=CH-CH_3$ | $^1$H-NMR*): 1,9 |
| 121 | 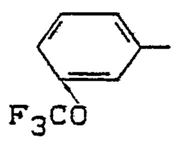 | CN | $=CH-C(CH_3)_3$ | $^1$H-NMR*): 1,1 |
| 122 | 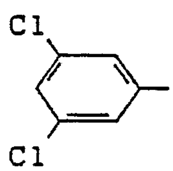 | CN | $=CH_2$ | 174-176 |
| 123 | 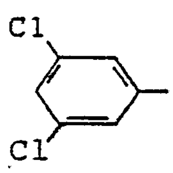 | CN | $=CH-C(CH_3)_3$ | $^1$H-NMR*): 1,12 |
| 124 | 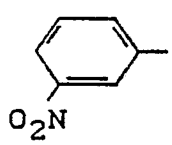 | CN | $=CH_2$ | 142 |

| Bsp.Nr. | $R^1$ | $R^2$ | 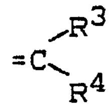 | Schmelz-punkt° C |
|---|---|---|---|---|
| 125 | 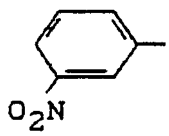 | CN | $=CH-CH_3$ | 93 |
| 126 | 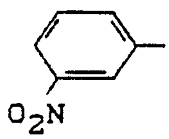 | CN | $=CH-C(CH_3)_3$ | 114-116 |
| 127 | 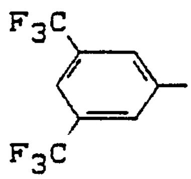 | CN | $=CH-CH_3$ | 96-98 |
| 128 | 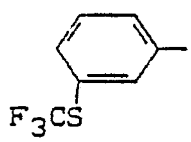 | CN | $=CH-CH_3$ | 67 |
| 129 | 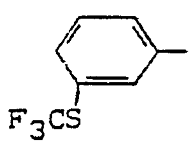 | CN | $=CH_2$ | 106-108 |
| 130 | 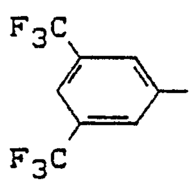 | CN | $=CH_2$ | 142-149 |
| 131 | 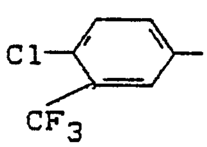 | CN | $=CH_2$ | $^1$H-NMR*):6,8 |
| 132 | 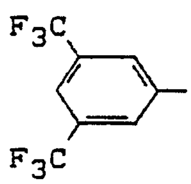 | CN | 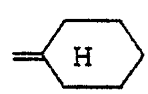 | $^1$H-NMR*):6,8 |

| Bsp.Nr. | $R^1$ | $R^2$ | 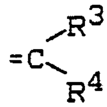 | Schmelz-punkt° C |
|---|---|---|---|---|
| 133 | 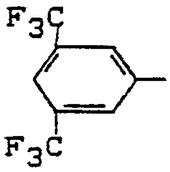 | CN | $=CH-C(CH_3)_3$ | $^1$H-NMR*): 6,7 |
| 134 | 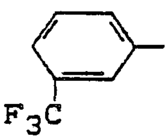 | CN | 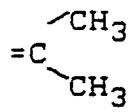 | $^1$H-NMR*): 6,75 |
| 135 | 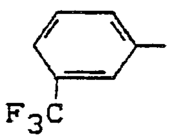 | CN | 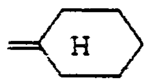 | $^1$H-NMR*): 6,75 |
| 136 | 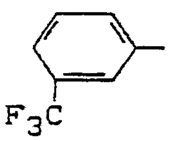 | CN | $=CH-C(CH_3)_3$ | $^1$H-NMR*): 6,7 |
| 137 | 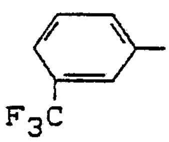 | CN | $=CH_2$ | $^1$H-NMR*): 6,8 |
| 138 | 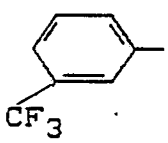 | CN | $=CH-CH_3$ | $^1$H-NMR*): 1,9 |
| 139 | 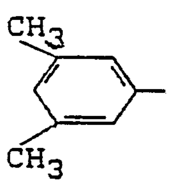 | CN | 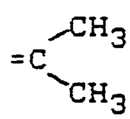 | $^1$H-NMR*): 6,75 |
| 140 | 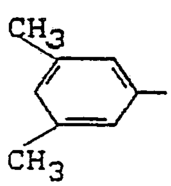 | CN | 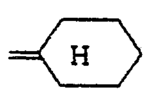 | $^1$H-NMR*): 6,75 |

| Bsp.Nr. | $R^1$ | $R^2$ | 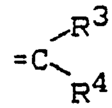 | Schmelzpunkt° C |
|---|---|---|---|---|
| 141 | 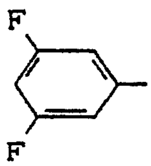 | CN | $=CH-CH_3$ | $^1H$-NMR*): 1,9 |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

<u>Anwendungsbeispiele:</u>

In dem folgenden Anwendungsbeispiel wurde die nachstehend aufgeführte Verbindung als Vergleichssubstanz eingesetzt:

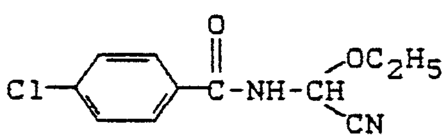

(A)

2-(4-Chlorbenzoylamino)-2-ethoxy-acetonitril (bekannt aus EP 59 536)

<u>Beispiel A</u>

Phytophthora-Test (Tomate) /protektiv

Lösungsmittel:4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20° C aufgestellt.

3 Tage nach der Inokulation erfolgt die Auswertung.

Die erfindungsgemäßen Verbindungen 1, 2, 4, 5, 6, 22 zeigen in diesem Test eine deutliche Überlegenheit in der Wirksamkeit gegenüber dem Stand der Technik.

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 4, 5, 6, 7, 10, 40 und 47.

Beispiel C

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25° C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 4, 5, 6, 7, 8, 22, 40 und 54.

Beispiel D

Tetranychus-Test (resistent)

Lösungsmittel: 7 Gewichtsteil Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen die meisten Verbindungen der Herstellungsbeispiele eine gute Wirksamkeit.

**Ansprüche**

1. Alkoximinoalkylamide der allgemeinen Formel (I),

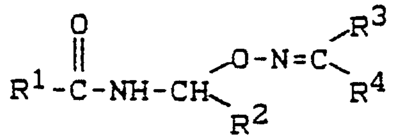

(I)

in welcher

$R^1$ für jeweils gegebenenfalls substituiertes Alkyl, Alkenyl, Aralkyl, Heteroarylalkyl, Aryl oder Heteroaryl steht,

$R^2$ für Cyano, für die Gruppe

$-\overset{O}{\overset{\|}{C}}-NH_2$ oder für die Gruppe

$-\overset{S}{\overset{\|}{C}}-NH_2$ steht und

$R^3$ und $R^4$ unabhängig voneinander jeweils für Wasserstoff, für gegebenenfalls substituiertes Alkyl, für Alkenyl oder für Alkinyl stehen, oder gemeinsam mit dem Kohlenstoffatom, an welches sie gebunden sind, für einen gesättigten carbocyclischen Ring stehen.

2. Alkoximinoalkylamide gemäß Anspruch 1, bei welchen in der allgemeinen Formel (I)

$R^1$ für geradkettiges oder verzeigtes Alkyl mit 1 bis 8 Kohlenstoffatomen, für geradkettiges oder verzweigtes Cyanalkyl mit 1 bis 5 Kohlenstoffatomen im Alkylteil, für geradkettiges oder verzweigtes Halogenalkyl mit 1 bis 5 Kohlenstoffatomen und 1 bis 9 Halogenatomen, für jeweils geradkettiges oder verzweigtes Alkoxyalkyl oder Alkylthioalkyl mit jeweils 1 bis 5 Kohlenstoffatomen in den einzelnen Alkylteilen, für geradkettiges oder verzweigtes Alkenyl mit 3 bis 8 Kohlenstoffatomen oder für geradkettiges oder verzweigtes Halogenalkenyl mit 3 bis 8 Kohlenstoffatomen und 1 bis 5 Halogenatomen steht, außerdem für im Arylteil gegebenenfalls einfach oder mehrfach, gleich oder verschieden substituiertes Aralkyl mit 1 bis 4 Kohlenstoffatomen im Alkylteil und 6 bis 10 Kohenstoffatomen im Arylteil, für im Heteroarylteil gegebenenfalls einfach oder mehrfach, gleich oder verschieden substituiertes Heteroarylalkyl mit 1 bis 4 Kohlenstoffatomen im Alkylteil und 2 bis 9 Kohlenstoffatomen sowie 1 bis 3 Heteroatomen -insbesondere Stickstoff, Sauerstoff und Schwefel -im Heteroarylteil, für gegebenenfalls einfach oder mehrfach, gleich oder verschieden substituiertes Aryl mit 6 bis 10 Kohlenstoffatomen oder für gegebenenfalls einfach oder mehrfach, gleich oder verschieden substituiertes Heteroaryl mit 2 bis 9 Kohlenstoffatomen und 1 bis 3 Heteroatomen -steht, wobei als Aryl bzw. Heteroarylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, Alkylendioxy mit 1 bis 3 Kohlenstoffatomen, Phenyl, Phenoxy, Formyl, Alkanoyl mit 1 bis 5 Kohlenstoffatomen, Alkoxycarbonyl mit 1 bis 5 Kohlenstoffatomen oder Alkoximinoalkyl mit jeweils 1 bis 5 Kohlenstoffatomen in den einzelnen Alkylteilen,

$R^2$ für Cyano, für die Gruppe

$-\overset{O}{\overset{\|}{C}}-NH_2$ oder für die Gruppe

$-\overset{S}{\overset{\|}{C}}-NH_2$ steht und

$R^3$ und $R^4$ unabhängig voneinander jeweils für Wasserstoff, für geradkettiges oder verzweigtes Alkyl mit 1 bis 8 Kohlenstoffatomen, für geradkettiges oder verzweigtes Cyanalkyl mit 1 bis 5 Kohlenstoffatomen, für geradkettiges oder verzweigtes Halogenalkyl mit 1 bis 5 Kohlenstoffatomen und 1 bis 9 Halogenatomen, für jeweils geradkettiges oder verzweigtes Alkoxyalkyl oder Alkylthioalkyl mit jeweils 1 bis 5 Kohlenstoffatomen in den einzelnen Alkylteilen oder für jeweils geradkettiges oder verzweigtes Alkenyl oder Alkinyl mit jeweils 3 bis 7 Kohlenstoffatomen stehen oder

$R^3$ und $R^4$ gemeinsam mit dem Kohlenstoffatomen, an welches sie gebunden sind, für einen gesättigten 5- bis 7-gliedrigen carbocyclischen Ring stehen.

3. Alkoximinoalkylamide gemäß Anspruch 1, bei welchen in der allgemeinen Formel (I)

$R^1$ für geradkettiges oder verzweigtes Alkyl mit 1 bis 5 Kohlenstoffatomen, für Methoxymethyl, für Methylthiomethyl, für Allyl, Butenyl, für jeweils gegebenenfalls ein-bis fünffach gleich oder verschieden im Arylteil substituiertes Benzyl, Phenylethyl, Phenyl oder Naphthyl steht oder für jeweils gegebenenfalls im Heteroarylteil ein-bis dreifach gleich oder verschieden substituiertes Heteroarylmethyl oder Heteroaryl steht, wobei als Heteroaromaten jeweils infrage kommen:

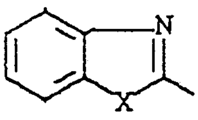 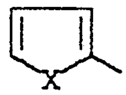 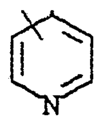 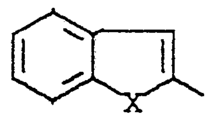 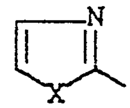

wobei X jeweils für Sauerstoff oder Schwefel steht und als Aryl-bzw. Heteroarylsubstituenten jeweils infrage kommen: Fluor, Chlor, Brom, Iod, Cyano, Nitro, Methyl, Ethyl, n-oder, i-Propyl, n-, i-, s-oder t-Butyl, Methoxy, Ethoxy, Methylthio, Trifluormethyl, Trifluormethoxy, Trifluormethylthio, Methylendioxy, Ethylendioxy, Phenyl, Phenoxy, Formyl, Acetyl, Methoxycarbonyl, Ethoxycarbonyl, Methoximinomethyl, Methoximinoethyl und Ethoximinomethyl,

$R^2$ für Cyano oder für eine Thiocarbamoylgruppe steht und

$R^3$ und $R^4$ unabhängig voneinander jeweils für Wasserstoff, für Methyl, Ethyl, n-oder i-Propyl, n-, i-, s-oder t-Butyl, für Allyl, Propenyl, n-oder i-Butenyl oder Propargyl stehen oder gemeinsam mit dem Kohlenstoffatom, an welches sie gebunden sind, für einen Cyclopentyl-oder Cyclohexylrest stehen.

4. Alkoximinoalkylamide gemäß Anspruch 1, bei welchen in der allgemeinen Formel (I)

$R^1$ für jeweils gegebenenfalls ein-bis dreifach, gleich oder verschieden substituiertes Phenyl, Pyridyl, Benzoxazolyl, Benzthiazolyl, Furyl oder Thienyl steht, wobei als Substituenten jeweils infrage kommen: Methyl, Ethyl, Methoxy, Fluor, Chlor, Brom, Methylendioxy oder Ethylendioxy,

$R^2$ für Cyano steht und

$R^3$ und $R^4$ unabhängig voneinander jeweils für Wasserstoff, Methyl oder Ethyl stehen.

5. Verfahren zur Herstellung von Alkoximinoalkylamiden der allgemeinen Formel (I),

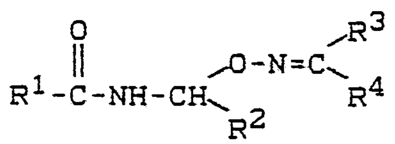

(I)

in welcher

$R^1$ für jeweils gegebenenfalls substituiertes Alkyl, Alkenyl, Aralkyl, Heteroarylalkyl, Aryl oder Heteroaryl steht,

$R^2$ für Cyano, für die Gruppe

$-\overset{O}{\overset{\|}{C}}-NH_2$ oder für die Gruppe

$-\overset{S}{\overset{\|}{C}}-NH_2$ steht und

$R^3$ und $R^4$ unabhängig voneinander jeweils für Wasserstoff, für gegebenenfalls substituiertes Alkyl, für Alkenyl oder für Alkinyl stehen, oder gemeinsam mit dem Kohlenstoffatom, an welches sie gebunden sind, für einen gesättigten carbocyclischen Ring stehen,

dadurch gekennzeichnet, daß man

(a) bromsubstituierte Amide der allgemeinen Formel (II),

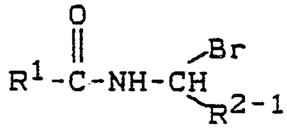

(II)

in welcher

$R^1$ die oben angegebene Bedeutung hat und

$R^{2-1}$ für Cyano oder für eine Carbamoylgruppe steht,

mit Oximen der allgemeinen Formel (III),

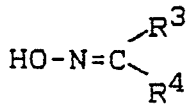

(III)

in welcher
$R^3$ und $R^4$ die oben angegebene Bedeutung haben,
gegebenenfalls in Gegenwart eines Verdünnungsmittels sowie gegebenenfalls in Gegenwart eines Säurebindemittels umsetzt und gegebenenfalls anschließend

(b) die nach Verfahren (a) erhältlichen Alkoximinoalkylamide der allgemeinen Formel (Ia),

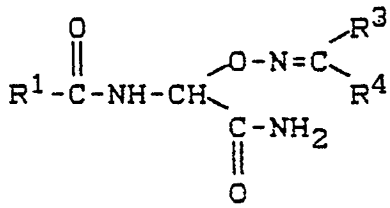

(Ia)

in welcher
$R^1$, $R^3$ und $R^4$ die oben angegebene Bedeutung haben,
gegebenenfalls in Gegenwart eines Verdünnungsmittels und gegebenenfalls in Gegenwart eines Säurebindemittels mit einem wasserabspaltenden Mittel dehydratisiert und gegebenenfalls anschließend

(c) die nach Verfahren (a) oder (b) erhältlichen Alkoximinoalkylamide der allgemeinen Formel (Ib),

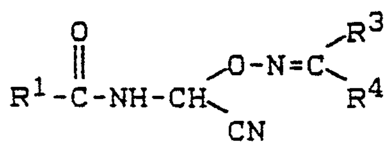

(Ib)

in welcher
$R^1$, $R^3$ und $R^4$ die oben angegebene Bedeutung haben,
mit Schwefelwasserstoff gegebenenfalls in Gegenwart eines Verdünnungsmittels und gegebenenfalls in Gegenwart eines basischen Katalysators umsetzt.

6. Schädlingsbekämpfungsmittel, gekennzeichnet durch einen Gehalt an mindestens einer Verbindung der allgemeinen Formel (I) gemäß Anspruch 1.

7. Verfahren zur Bekämpfung von pilzlichen und tierischen Schädlingen, dadurch gekennzeichnet, daß man mindestens eine Verbindung der Formel (I) gemäß Anspruch 1 auf pilzliche oder tierische Schädlinge und/oder ihren Lebensraum einwirken läßt.

8. Verwendung von Verbindungen der allgemeinen Formel (I) gemäß Anspruch 1 zur Bekämpfung von pilzlichen Schädlingen.

9. Verwendung von Verbindungen der allgemeinen Formel (I) gemäß Anspruch 1 zur Bekämpfung von tierischen Schädlingen.

10. Verfahren zur Herstellung von Schädlingsbekämpfungsmitteln, dadurch gekennzeichnet, daß man Verbindungen der allgemeinen Formel (I) gemäß Anspruch 1 mit Streckmitteln und/oder oberflächenaktiven Mitteln vermischt.